# EUROPEAN PATENT APPLICATION

(11) **EP 4 079 234 A1**
(43) Date of publication of application: **26.10.2022**
(21) Application number: 21315070.9
(22) Date of filing: 23.04.2021
(51) Int. Cl.: A61B 17/11, A61B 17/00

(54) **APPARATUS FOR FORMING AN ANASTOMOSIS**

(71) Applicant: BariaTek Medical, 75003 Paris (FR)
(72) Inventor: BIADILLAH, Joussef, 78600 Maisons-Laffitte (FR); SEJOR, Eric, 06000 Nice (FR)
(74) Representative: Hepp Wenger Ryffel AG

(57) **Abstract**

Apparatus comprising a stent (1) expandable from a non-deployed configuration for delivery to a target site for an anastomosis, and a deployed configuration for forming a temporary stented-anastomosis (201) with a through-channel. Magnetic attraction between first and second portions (11, 12) around the through-channel induces formation of.a permanent, magnetic compression anastomosis (202) to biologically supplant the temporary stent-ed-anastomosis (201). The apparatus may especially suitable for use in the digestive system, optionally in the gastrointestinal tract.

## Description

The present invention relates to the field of anastomosis formation between body lumina. In one non-limiting aspect, the invention relates to forming an anastomosis in a patient's digestive system, preferably in the gastrointestinal tract.

An anastomosis is a surgical cross-connection or bridge between two different sections of body lumen. The gastrointestinal tract is the luminal route in the body from the esophagus to the anus. Anastomoses formed somewhere along or in the gastrointestinal tract are one form of therapy used to treat digestion-related problems, such as diabetes, obesity, bowel diseases and obstructions. Although an anastomosis can be formed by an open-surgery procedure, significant technical challenges remain in achieving anastomoses equally effectively by minimally invasive procedures, for example, laparoscopically or endoscopically.

It would be desirable to address and/or mitigate such problems, and/or facilitate minimally invasive anastomosis formation.

Aspects of the invention are defined in the claims.

Additionally or alternatively, one aspect of the invention provides apparatus for forming an anastomosis of two body lumen wall sections, optionally of the digestive system, optionally of the gastrointestinal tract.

The apparatus has a non-deployed configuration for introduction to a target site for the anastomosis, and a deployed configuration for implantation.

The apparatus comprises a stent expandable from the non-deployed configuration to the deployed configuration for forming a stented anastomosis. In the deployed configuration, the stent is anchored with respect to the wall sections, and/or provides a through-channel, for example, for communication of body fluids between the two body lumen sections.

In the deployed configuration of the apparatus, first and second portions of the apparatus are disposed laterally outside the through-channel in an annular arrangement. The first and second portions are magnetically attracted to each other, for forming a compression anastomosis around the through-channel of the stent.

Additionally or alternatively to the above first aspect, a second aspect of the invention provides apparatus for forming an anastomosis of two body lumen wall sections, optionally of the digestive system, optionally of the gastrointestinal tract. The apparatus comprises a stent expandable from a non-deployed configuration for introduction to a target site for the anastomosis, to a deployed configuration for implantation to form a stented-anastomosis. In the deployed condition, the stent is anchored with respect to the wall sections, and/or provides a through-channel, for example, for communication of body fluids between the two body lumen sections;

In the deployed configuration of the stent, the apparatus is also configured to apply pressure by magnetic attraction between first and second portions of the apparatus, to induce formation of a permanent compression anastomosis enclosing and laterally outward of through-channel of the stent. For example, the first and second portions are disposed laterally outwardly of the through-channel of the stent. The first and second portions may enclose the periphery of the through-channel, optionally at opposite ends of the through-channel.

Additionally or alternatively to the first and/or second aspect above, a third aspect of the invention provides apparatus for forming an anastomosis of two body lumen wall sections, optionally of the digestive system, optionally of the gastrointestinal tract. The apparatus comprises an expandable stent with a through channel. The apparatus is configured to form a stented-anastomosis at target site, and to form a compression anastomosis by magnetic compression of wall tissue around the stented-anastomosis.

For example, the stent is expandable from a non-deployed configuration for introduction to the target site for the anastomosis, and a deployed configuration for implantation to form the stented anastomosis. In the deployed configuration, the apparatus is further configured to induce the compression anastomosis, via magnetic attraction between first and second portions of the apparatus, around the stented-anastomosis.

Additionally or alternatively to the first and/or second and/or third aspect, a fourth aspect of the invention provides apparatus for forming a magnetic compression anastomosis of two body lumen wall sections, optionally of the digestive system, optionally of the gastrointestinal tract. The apparatus comprises a first portion and a second portion magnetically attracted to the first portion for inducing a compression anastomosis in a region of the wall sections. The apparatus comprises or further comprises an expandable stent for providing a stented-anastomosis within said region of the wall sections, for defining a temporary through-channel until completion of the compression anastomosis.

With each or any of the above aspects, in use, the (e.g. permanent) compression anastomosis induced by the magnetic attraction can biologically supplant the stented anastomosis.

The apparatus has the advantage of being able to provide a working anastomosis immediately on implantation, by means of the expandable stent, and additionally induce formation in the body of a permanent compression anastomosis. The stent may be regarded as providing an initial or temporary anastomosis. Upon implantation, a medical practitioner can verify correct placement and functioning of the stented anastomosis. The stented anastomosis can also provide immediate relief and/or therapy for a medical condition to be treated. The stent itself can provide a well-defined, self-supporting through-channel, unaffected by risk of narrowing due to tissue growth and healing around the anastomosis. The stent does not need to provide a durable anastomosis, because the compression anastomosis forms around the stent, typically within a period of a few weeks or months (e.g. typically 3-4 weeks). Not requiring the stent to be highly durable provides considerable design freedom and cost benefits. The compression anastomosis forms by necrosis of the compressed tissue of the two wall sections pressed together by the magnetic attraction, and tissue healing around the necrosed tissue resulting in a unitary and/or permanent join. Once healing has reached a suitable stage, the necrosed tissue detaches from the surrounding tissue walls, such that the necrosed tissue and the attached stent can move away, e.g. naturally or aided by a subsequent procedure to detach and/or extract and/or retrieve the apparatus.

By way of example, the target site for the anastomosis may be between a wall section of the stomach and a wall section of the jejunum, for forming a gastro-jejunal anastomosis bypassing the duodenum and optionally a part of the jejunum. An alternative target site example is between a wall section of the ileum and a wall section of the jejunum, for forming an ileo-jejunal anastomosis.

Use of expandable apparatus and/or an expandable stent enables the installation procedure to be carried out minimally invasively, optionally laparoscopically and/or endoscopically.

The stent may be self-expanding from the non-deployed configuration to the deployed configuration, and/or it may be forcibly expanded (for example, by an expansion balloon or other deployment device applying deploying forces to the stent). A self-expanding stent may, for example, be made of or comprise shape memory material, for example, a shape memory metal alloy and/or shape memory plastics. Suitable alloys may comprise nickel and titanium (e.g. NiTi alloy and/or Nitinol), optionally in combination with cobalt (e.g. NiTiCo). Whatever the material, in the deployed configuration, the stent preferably provides a self-supporting structure, optionally a frame-like and/or lattice-like and/or mesh-like structure. The stent may be monolithic or made from one or more elements coupled together directly (e.g. by at least one connector or fixing) or indirectly (e.g. by braiding or weaving).

At least a portion of the stent (e.g. a connector) may be made of material that weakens with time, for example bioresorbable material, such that at least a portion of the stent loses strength. The stent may be configured to change configuration from the deployed configuration to a post-deployed configuration. For example, when a portion of the stent weakens, the stent itself or another member (such as an elastic cover) of the apparatus may deform the stent to the post-deployed configuration. In the post-deployed configuration, the apparatus may have, at least partly, a more streamlined shape to facilitate passage of the apparatus through, for example, the bowel to be excreted. For example, the post-deployed configuration may be elongate, or oval or lozenge-shaped.

In some embodiments, the (e.g. smallest) transverse dimension (e.g. diameter) of the through-channel when the stent is in the deployed configuration is larger than the transverse dimension (e.g. diameter) of the stent and/or of the apparatus when in the non-deployed configuration.

The magnetic attraction may be provided by one or more magnets, optionally carried by or embodied within the stent. Providing one or more magnets with the stent enables the magnets and stent to be introduced together, for example, using a single delivery device. The magnets may be or comprise a plurality of individual magnets carried by the stent. The magnets may be provided in such a way to permit the stent to be collapsed to its non-deployed configuration, and expanded to its deployed configuration. In some embodiments, the magnets may be spaced apart around a periphery of the stent, for example, spaced apart by a circumferential distance at least equal to a circumferential (or tangential) dimension of the magnet. In other embodiments, the magnets may provide a more continuous or substantially continuous arrangement, for example, such that the spacing between adjacent magnets is less than the circumferential (or tangential) dimension of the magnets.

In some embodiments, the stent carries a pressure element, for example a flexible filament adjacent to the magnets. The flexible filament, or other pressure element, may be configured to provide a substantially closed loop shape, optionally in combination with or collectively with the magnets, such that pressure is applied to body tissue around a continuous closed-loop. The flexibility of the filament permits the stent to be collapsed to the non-deployed configuration, and expanded to the deployed configuration. The filament may be made of, or comprise, a polymer and/or a shape memory material (for example, a shape memory alloy as described above).

In some embodiments, the stent and/or a cover of the stent, is configured to provide a generally atraumatic exterior surface or shape, when in the deployed (and/or post-deployed) configuration, for facilitating passage of the apparatus out of the body, for example, when excreted via the anus. For example, the apparatus may have a generally rounded toroid shape. Any abrupt edges of the apparatus and/or stent may be configured to face an interior of the structure. Additional struts may be provided to bridge over portions of the stent that have abrupt contour.

A further aspect of the present invention provides a method of forming an anastomosis at a target site, optionally using an apparatus as described above in any aspect, the method comprising the steps of:
- introducing an expandable stent to the target site;
- expanding the stent from a non-deployed configuration to a deployed configuration forming a temporary stented anastomosis between tissue wall sections at the target site, the stent having a through-channel to provide a functional anastomosis; and
- simultaneously or non-simultaneously with the expanding step, positioning first and second portions of the apparatus against tissue of the wall sections around the through-channel, the first and second portions magnetically attracting each other, for inducing formation of a magnetic compression anastomosis around the through-channel of the stent.

Non-limiting embodiments of the invention are now described, by way of example only, with reference to the accompanying schematic drawings, in which:
- Fig. 1: is a side view of a stent with magnets, with the stent depicted in an as-cut, rolled out style prior to heat shaping;
- Fig. 2: is a cross-section of the stent in the configuration of Fig. 1. This configuration corresponds to the non-deployed configuration, except that the non-deployed configuration may be compressed more radially. The wider illustration of Fig. 2 is presented for ease of viewing the apparatus features;
- Fig. 3: is a cross-section of the stent of Figs. 1 and 2 in a deployed configuration;
- Fig. 4: is a sectional view illustrating endoscopic insertion of a delivery device into a patient to a target site for an anastomosis in the gastrointestinal tract;
- Fig. 5: is a sectional view similar to Fig. 4 illustrating a next step of puncturing the tissue walls of the gastrointestinal tract at the target site;
- Fig. 6: is a sectional view similar to Fig. 5 illustrating a next step of retracting the delivery device towards the apertures of the two tissue walls;
- Fig. 7: shows a larger depiction of the distal end of the delivery device shown in Fig. 6;
- Fig. 8: is a sectional view similar to Fig. 6 illustrating a next step of partly deploying the stent with magnets in proximity of the distal-most tissue wall aperture;
- Fig. 9: shows a larger depiction of the distal end of the delivery device and partly deployed stent of Fig. 8;
- Fig. 10: is a sectional view similar to Fig. 8 illustrating a next step of further retracting the delivery device while deploying the stent with magnets;
- Fig. 11: shows a larger depiction of the distal end of the delivery device and stent of Fig. 10;
- Fig. 12: is a sectional view similar to Fig. 10 showing a next step of releasing the stent with magnets to its deployed configuration to apply magnetic compression to the tissue walls while also providing a temporary stented anastomosis with a working through-channel;
- Fig. 13: shows a larger depiction of the stent of Fig. 12 in its deployed configuration;
- Fig. 14: is a sectional view similar to Fig. 12 showing a next step in which a permanent second, compression anastomosis forms around the stented-anastomosis;
- Fig. 15: shows a larger depiction of formation of the compression anastomosis of Fig. 14;
- Fig. 16: is a sectional view similar to Fig. 14 illustrating a next step in which the stented-anastomosis is supplanted by the compression anastomosis;
- Fig. 17: shows a larger depiction of the supplanting of the stented-anastomosis of Fig. 16;
- Fig. 18: is a side view of a second example of stent with magnets, with the stent depicted in an as-cut rolled-out style in its non-deployed configuration;
- Fig. 19: is a side view of the stent of Fig. 18, depicted in an as-cut rolled out style, in its deployed configuration;
- Fig. 20: is an end view of the stent of Figs. 18 and 19 in its deployed configuration;
- Fig. 21: is a sectional view of a further example of stent with magnets in its deployed configuration;
- Fig. 22: is a sectional view of a further example of stent with magnets in its deployed configuration;
- Fig. 23: is a sectional view of a further example of stent with magnets in its deployed configuration;
- Fig. 24: is a top view of an alternative embodiment of the stent with magnets in its deployed configuration;
- Fig. 25: is a section view similar to Fig. 13, showing a modified mounting of magnets with respect to the stent.
- Fig. 26: is an end view similar to Fig. 24, but showing an alternative stent shape.
- Fig. 27: is a profile view illustrating the stent changing shape from a generally round deployment configuration to a generally streamlines post-deployment configuration.
- Fig. 28: is a perspective view of a stent for creating an anastomosis between the stomach and bowel in a first comparative example;
- Figs. 29 and 30: are sectional views showing two magnetic members for forming an anastomosis in a second comparative example.

In the following description, the same reference numerals are used to denote corresponding features whether or not described explicitly. Features from one embodiment may be used in combination with another, whether or not described explicitly.

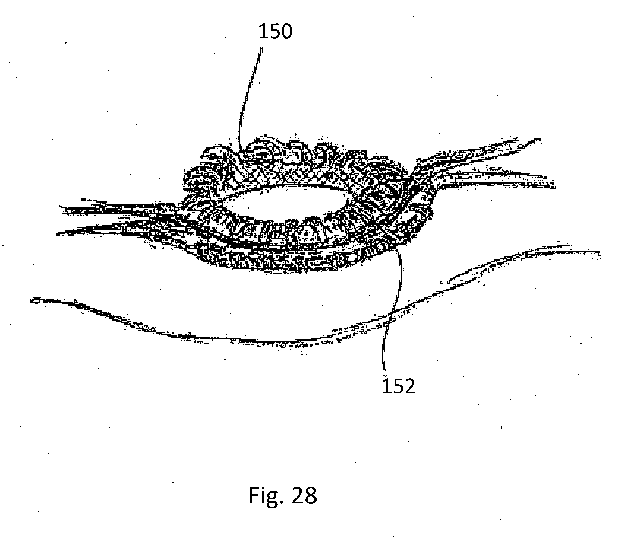

Before discussing the invention, reference is made to Figs. 28 to 30 which show two comparative examples, so that advantages of the invention can be appreciated. Fig. 28 illustrates a first comparative example in the form of an expandable stent 150 implantable to provide a stented anastomosis 152 between two body lumina, such as the stomach and the jejunum. The junction between the tissue walls relies permanently on the stent 150 for integrity, and so durability of the anastomosis depends on the ability of the stent 150 to maintain a leak-free junction. Even with a complex stent design, the durability may be limited. For example, over time, the stent 150 may suffer from corrosive effects of digestive fluids, and from constant movement between the body lumina. Leakage easily occurs should the stent dislodge or break, or should any part of the junction become imperfect. Leakage may be life-threatening for the patient.

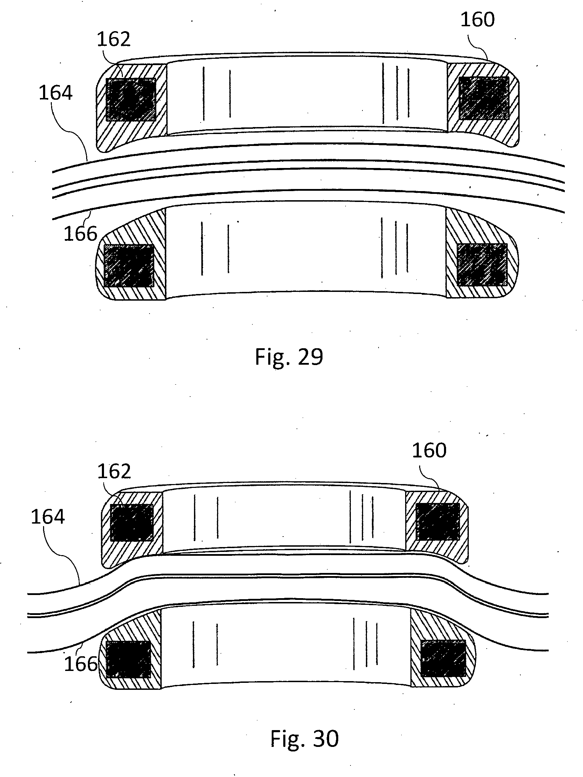

Figs. 29 and 30 illustrate a second comparative example in the form of a magnetic compression anastomosis formed by two rings 160 containing magnets 162. The two rings 160 are introduced separately using, for example, two separate endoscopes (not shown) to position the rings in contact with the tissue walls 164 and 166 such that the rings 160 are held by mutual attraction of the magnets 162. The anastomosis does not form immediately. Over time, say 3-4 weeks, necrosis of the tissue clamped between the rings 160 causes the surrounding tissue to unite and form a permanent tissue-tissue join. The necrotic tissue and the attached rings 160 eventually break loose, leaving behind a permanent anastomosis. Compared to the first comparative example, such a compression anastomosis has a potential advantage of being permanently leak free, and without depending on a permanent implant. However, unlike the first comparative example, the compression anastomosis is not immediate and requires sufficient time to form, which may vary from one patient to another. A medical practitioner does not know at the outset whether the anastomosis will be effective, because this can only be observed later, requiring a further investigative medical procedure, once the anastomosis has taken time to form by the rings breaking loose. The absence of an immediate working anastomosis means that a patient's medical condition cannot be treated at the outset. If, for some reason, the rings and necrotic tissue do not break loose, the anastomosis cannot function properly. Also, the procedure for introducing and positioning the two rings 160 on opposite sides of the two tissue walls 164 and 166 is more complicated in that two different endoscopes have to be used, and the endoscopes guided via different routes into alignment in the different body lumina.

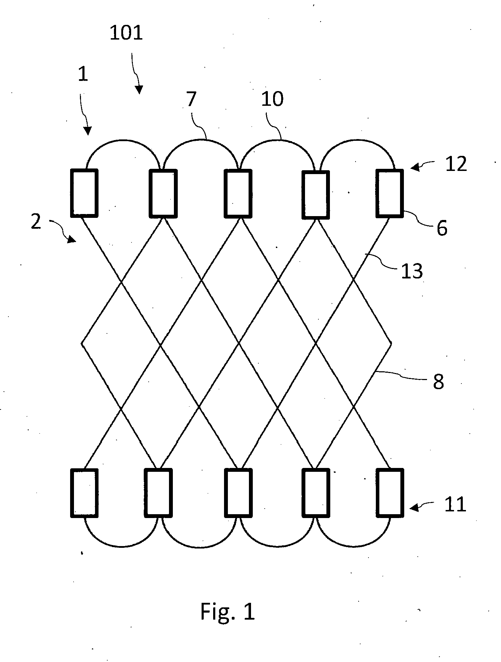

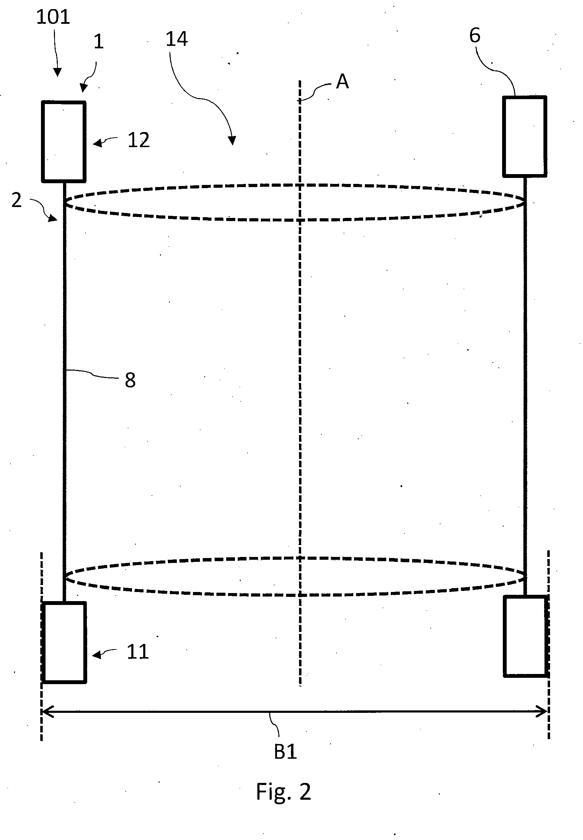

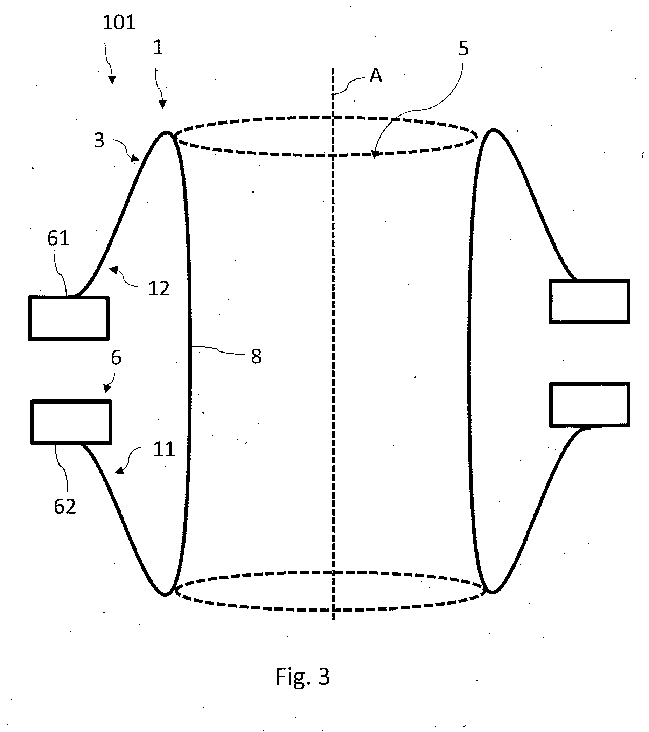

Figs. 1 to 27 illustrate embodiments of apparatus 101 according to the invention. Figure 1 shows a lateral view of an embodiment of an expandable stent 1 in an as-cut, rolled-out form prior to heat shaping. The stent 1 comprises a central tube region 8, and first and second portions 11 and 12 at opposite extremities carrying magnets 6 (61, 62, respectively). The stent is expandable from a non-deployed configuration (Fig. 2) for introduction to a target site for the anastomosis, to a deployed condition (Fig. 3) for implantation at the target site. In the non-deployed configuration (Fig. 2), the stent 1 is radially compressed with the first and second portions 11 and 12 at the extremities. The stent has a lateral dimension (e.g. diameter) B1, for example, in the range of 0.3 to 1.5 cm. In the deployed configuration (Fig. 3), the central tube region 8 expands radially to a lateral dimension larger than B1, and the first and second portions 11 and 12 bend outwardly and towards one another to define clamping elements around the central tube region 8. The first and second portions 11 and 12 may bend or articulate from opposite ends of the central tube region 8, such that the first and second portions 11 and 12 (and the magnets 6) move laterally outwardly and/or move closer together axially (in a longitudinal direction of the axis A). The central tube region 8 provides a through-channel from one end of the device to the other, having a lateral dimension (e.g. diameter) W1. For example, the dimension W1 may be in the range of about 1.5cm to 6cm.

The stent 1 may be a self-expanding type made of, or comprising, shape memory material, for example, nitinol. Such a stent 1 has to be retained in the non-deployed configuration by, for example, a constraining sheath, and self-expands progressively from one end as the sheath is slid off the stent and/or the stent is pushed out of the constraining sheath. For example, firstly one of the first and second portions 11, 12 may self-expand, followed by the central tube region 8, and finally the other of the first and second portions 11, 12. The stent 1 may have a frame structure to facilitate expansion. Some embodiments of stent 1 may be monolithic, for example, laser-cut from tubular stock. Alternatively, the stent 1 may be a meshed or braided structure.

The magnets 6 are arranged and/or orientated such the first and second portions 11 and 12 are magnetically attracted to each other. The attraction force is especially strong in the deployed configuration (Fig. 3) when the distance between the first and second portions 11 and 12 is reduced. Optionally, the magnets 6 within each portion 11, 12 may be arranged and/or orientated to repel each other, thus giving the respective portion 11, 12 an additional tendency to expand laterally and/or circumferentially from the non-deployed configuration. The magnets 6 are arranged in annular arrangements. In this embodiment, each magnet is mounted at an apex of the polygon structure. The discrete nature of the magnets leaves space between circumferentially adjacent magnets, notably in the deployed configuration. In the illustrated form, the magnets 6 are arranged in single rows, and each magnet in the first portion 11 is generally in register with a respective magnet in the section portion 12. Magnets 6 arranged offset to each other and/or several rows of magnets 6 in each portion 11, 12 are also conceivable.

At least one, and preferably both, of the first and second portions 11, 12 further comprises a pressure element 7 (best seen in Fig. 1) having or presenting, optionally collectively with the magnets 6, a closed-loop shape. The pressure element 7 may be a single element or it may comprise plural segments coupled together directly or indirectly. The pressure element 7 (optionally in combination with the magnets 6) serves to provide a substantially circumferentially continuous surface for applying pressure evenly to wall tissue around the closed loop, despite the individual magnets being circumferentially spaced apart, or discontinuous, in the deployed configuration. The pressure element 7 may be provided by a flexible filament, for example, a polymer filament. As illustrated in Fig. 1, the flexibility avoids the pressure element 7 obstructing compression of the stent 1 to the non-deployed configuration. The pressing element 7 may be pulled taught when in the deployed configuration.

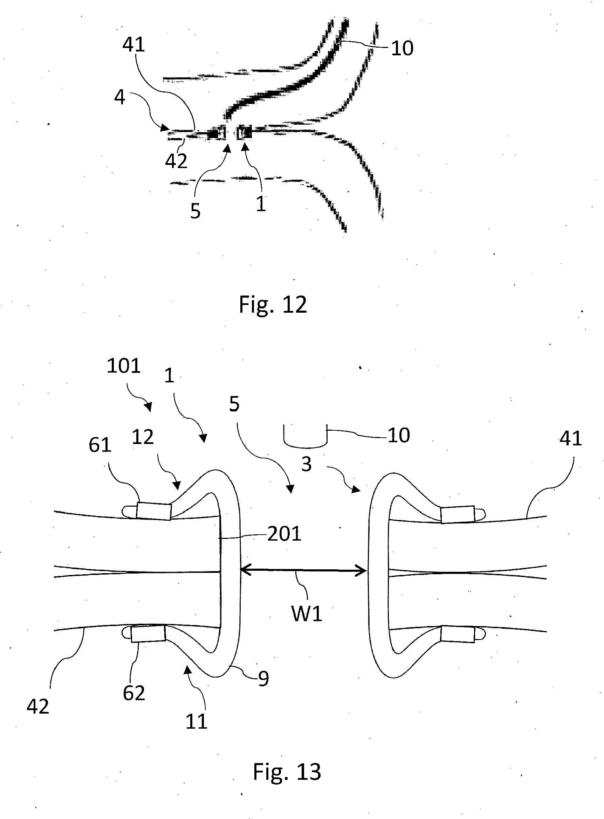

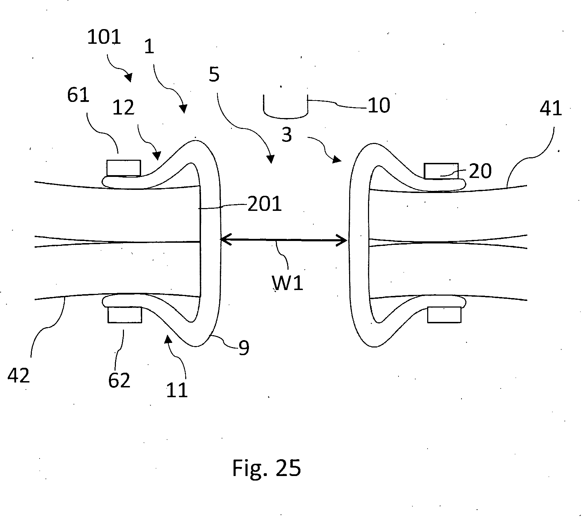

The magnets 6 (61, 62) may be generally coplanar with the portion of the stent 1 carrying the magnets 6 (as depicted schematically in Fig. 13), or the magnets 6 may in the deployed configuration lie closer to the tissue than that portion of the stent 1 (as depicted schematically in Fig. 3), or the magnets 6 may in the deployed configuration lie further from the tissue than that portion of the stent 1 (as depicted schematically in Fig. 25). In the latter case, the magnets may tend to compress the respective portion of the stent against the tissue, such that the stent struts contacting the tissue can also act as a pressure element for distributing the compression force around a closed loop shape.

The apparatus 101 and/or stent 1 can further comprise a flexible cover (not shown) covering at least partly a surface of the first region 11, and/or at least partly a surface of the central tube region 8, and/or at least partly a surface of the second region 12. The cover may optionally be generally impermeable to material passing through the body lumina for the anastomosis. The cover may be an interior and/or exterior cover. The cover may, for example, be or comprise a woven or non-woven synthetic fabric. The cover may serve to aid sealing of the stent with respect to the tissue walls and/or with respect to the through-channel, to reduce risk of leakage when the stent is deployed. Additionally or alternatively, the cover may serve to provide an atraumatic exterior of the apparatus, especially when in the deployed configuration.

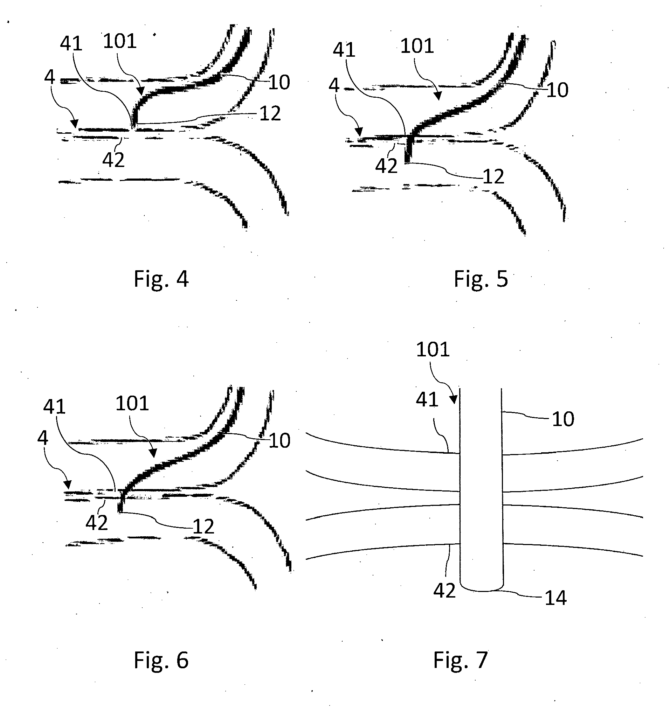

Figs. 4 to 17 illustrate a procedure for implanting the apparatus 101 of Figs. 1 to 3 using an endoscopic tool 10. Figure 4 illustrates introduction of the endoscopic tool 10 into the gastrointestinal tract of a patient to a target site for an anastomosis. For example, the target site may be between a wall section of the stomach and a wall section of the jejunum, for forming a gastro-jejunal anastomosis bypassing the duodenum and optionally a part of the jejunum. An alternative target site example is between a wall section of the ileum and a wall section of the jejunum, for forming an ileo-jejunal anastomosis.

The endoscopic tool 10 is insertable through the oesophagus of the patient. The tool 10 comprises cutting and/or piercing elements 12 which allow for puncturing the tissue walls 4 (41, 42, respectively) of the gastrointestinal tract, in particular the stomach and bowel. The piercing and/or cutting elements 12 are preferably arranged on, or are advanceable to, the distal region of the tool 10 allowing the tool 10 to penetrate through the tissue walls 41, 42 (Fig. 5).

Figures 6 and 7 illustrate retraction of the piercing and/or cutting elements 12, and optionally partial retraction of the tool 10 such that the distal end is positioned close to the apertures formed in the tissue walls 4. The endoscopic tool 10 optionally comprises one or more elements for bringing two tissue walls 41 and 42 into close contact. A partially deployed stent (illustrated in the next step) could also be used as such an element to bring the tissue walls 41, 42 into close contact.

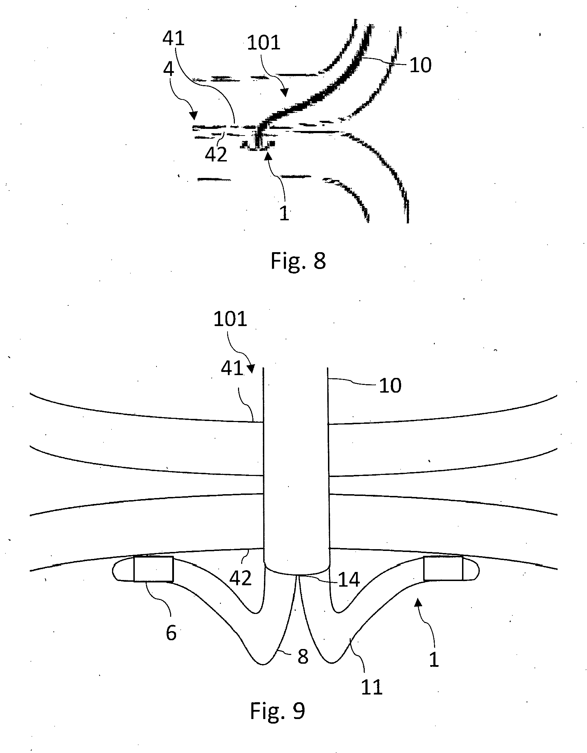

Figures 8 and 9 illustrate a first step of deployment of the apparatus/stent 1 for establishing an anastomosis. In the first step, the stent 1 is partly deployed by unsheathing one of the first and second portions, for example, the first portion 11, allowing the respective portion to self-expand. The stent 1 may be unsheathed by progressive retraction of a constraining sheath or by advancing the stent 1 progressively through the open end of a sheath. The first portion 11 begins to deploy by bending or moving towards its laterally extending position of the deployed configuration.

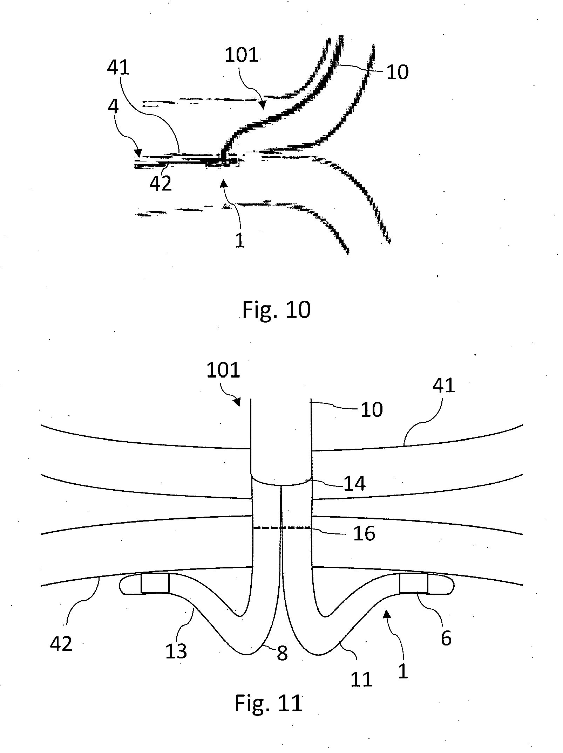

Figures 10 and 11 illustrate a next step of unsheathing the central tube region 8. As illustrated in the Fig. 11, the central tube region 8 may remain compressed until the stent as a whole is unrestrained. The stent 1 is prevented from being retracted through the tissue walls 41, 42 aperture by the increased maximum lateral profile.

Figures 12 and 13 illustrate complete unsheathing of the stent 1, releasing the second portion 12, and allowing the stent 1 to self-expand to its deployed configuration. The magnetic attraction between the magnets of the first and second portions 11 and 12 clamps the tissue walls 41 and 42 tightly together. The first and second portions 11 and 12 also serve to anchor the stent 1 in its position bridging the apertures in the tissue walls 41 and 42. The central tube portion 8, expands to its full width W1 and provides a through-channel 5 from one side of the tissue walls to the other. The stent 1 thus provides a stented-anastomosis 201 with a functional through-channel 5 immediately upon implantation of the apparatus. The lateral forces exerted by the central tubular portion 8 on the edges of the tissue walls, optionally aided by the flexible cover (described above) provides a seal to prevent leakage of stomach and/or bowel content from the stented-anastomosis 201.

The functional through-channel 5 can thus enable the stented-anastomosis to function and provide treatment or therapy of the patient's medical condition immediately upon implantation. The medical practitioner can observe the functioning and positioning of the stent-anastomosis to verify correct placement. Once the stent 1 has been placed, the endoscopic tool 10 can be withdrawn and optionally removed from the body.

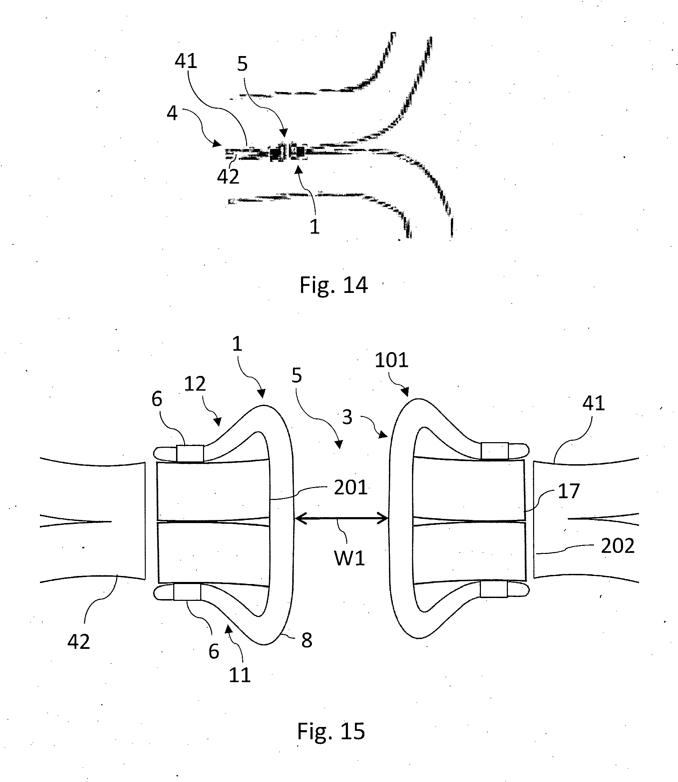

Referring to Figs. 14 and 15, the magnetic attraction between the first and second portions 11 and 12 arranged around the through-channel induces formation of a magnetic compression anastomosis. The magnetic attraction applies pressure to the tissue walls 41, 42 restricting blood perfusion in the clamped regions of the tissue walls. Over time, typically around 3-4 weeks, the clamped tissue becomes necrotic (indicated at 17), inducing the surrounding tissue to heal as a permanent tissue-tissue join, from which the necrotic tissue separates or breaks off naturally.

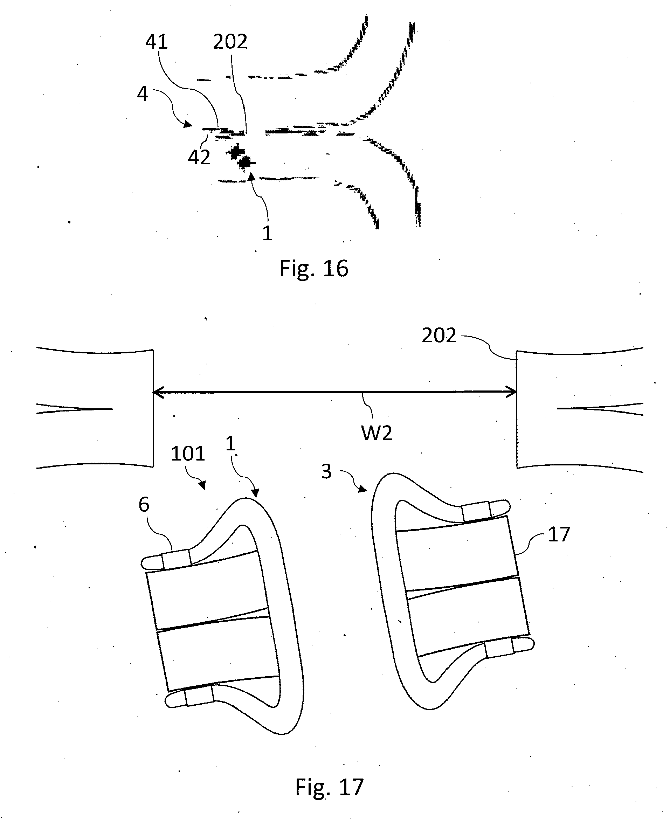

Referring to Figs. 16 and 17, the stent 1 and/or apparatus 101 is attached only to the necrotic tissue 17. Separation of the necrotic tissue 17 results in the stent 1 moving away from the tissue walls 41, 42, leaving behind a permanent compression anastomosis 202, having a width W2 slightly wider than the width W1 of the channel of the stented-anastomosis 201. For example, the width W2 of the permanent anastomosis may be from about 2cm to about 6cm. The apparatus 101, still attached to the necrotic tissue, can pass through the digestive tract, and be excreted from the body.

The apparatus 101 thus allows creation of a temporary stented-anastomosis 201 that allows immediate function, and subsequently a permanent compression anastomosis 202 that biologically supplants the stented-anastomosis 201. The apparatus 101 can be implanted using a single minimally invasive surgical procedure, typically an endoscopic procedure, but optionally also a laparoscopic procedure.

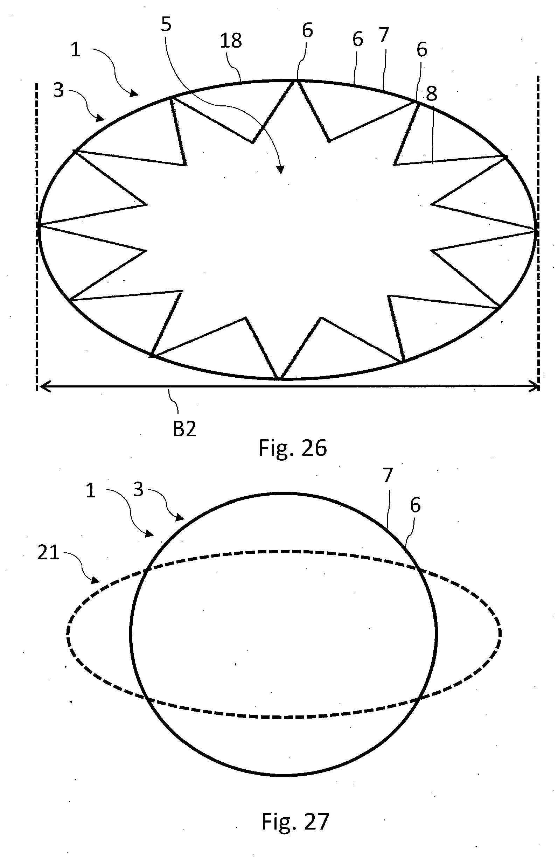

Optionally, the stent 1 can comprise breaking points and/or bioresorbable material so that the dimension can be decreased by the stent 1 detaching into separate segments (not shown in Fig. 17). One implementation of the stent 1 comprises bioresorbable connectors or hinges which divide the stent 1 into segments so that the stent 1 can be excreted more easily. An alternative embodiment of the stent 1 comprising breaking points can be conceived to break once the magnets 6 were brought into close contact after successful creation of a permanent anastomosis 202. A further embodiment may transform in shape to a post-implantation configuration (Fig. 27). For example, one or more structural support portions of the stent may weaken, optionally break, allowing force applied by another portion of the apparatus to deform the stent to its post-implantation position. For example, the force may come from the stent itself, or from an elastic cover. In the post-deployment configuration, the shape of the apparatus may be more streamlined than in the deployment configuration, for example slightly elongate, to facilitate passage through the bowel and through the anus. For example, Fig. 27 illustrates the stent 1 being transformed from a generally round deployment configuration to a generally elongate (e.g. oval, or elliptical or lozenge shape) post-deployment configuration or profile 21.

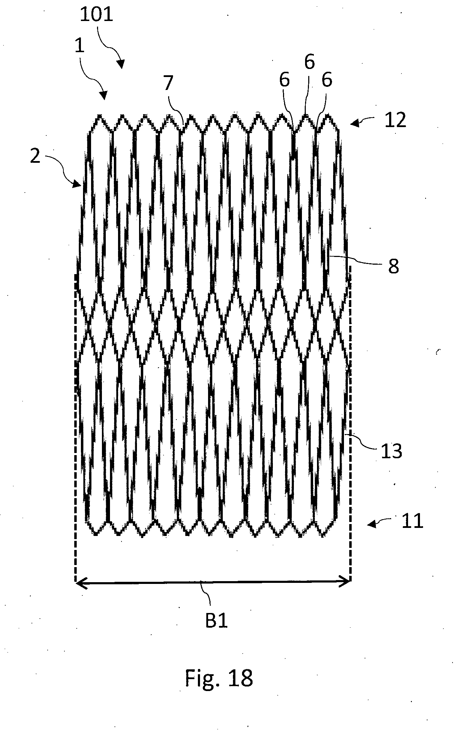

Figure 18 shows a side view of a modified embodiment of a stent 1 with magnets 6 in the non-deployed configuration 2 of the apparatus 101. The stent 1 comprises a monolithic frame structure, defining 8. The shaping structure 8 comprises a polygon mesh which is materially connected at every crossing of the stabilizing elements 13. However, stabilizing elements 13 which are movable relative to each other could also be conceived. The magnets 6 are connected to the stent 1 in the distal region 11 and proximal region 12 of the stent 1. The magnets 6 are connected via the connecting elements 7. The magnets 6 are arranged on the stent 1 in the non-deployed configuration 2 in several rows that are offset in a longitudinal direction of the stent 1. The rows of magnets 6 are connected in a zig zag pattern of connecting elements 7 on the distal region 11 and proximal region 12. Several rows of magnets 6. allow for decreased attraction of neighbouring magnets 6 due to an increase in distance. The stent 1 can also comprise an impermeable structure (not shown in Fig. 18). The lateral profile width B1 of the stent 1 in the non-deployed configuration 2 is smaller than the lateral width of a catheter of the apparatus 101 (not shown in Fig. 18).

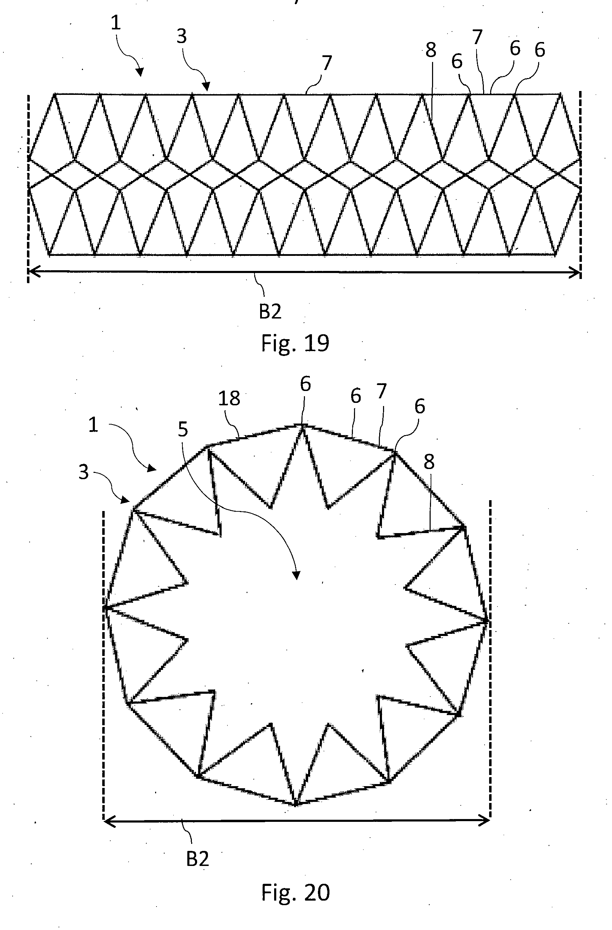

Figure 19 shows an embodiment of the stent 1 with magnets 6 according to Fig. 18 in the deployed configuration 3. In the deployed configuration 3 the magnets 6 which were arranged in separate rows in the non-deployed configuration are arranged on the closed loop equidistant from one another distanced by the connecting elements 7. The stent 1 assumes its deployed configuration 3 by creating a radial force increasing its lateral profile and expanding the temporary channel without external forces acting on the stent 1 (not shown in Fig. 19). The lateral profile width B2 of the stent 1 in the deployed configuration 3 is larger than the width of a catheter of the application 101 (not shown in Fig. 18). The lateral profile width B2 is also larger than the incision aperture of the tissue walls from the insertion of the apparatus 101.

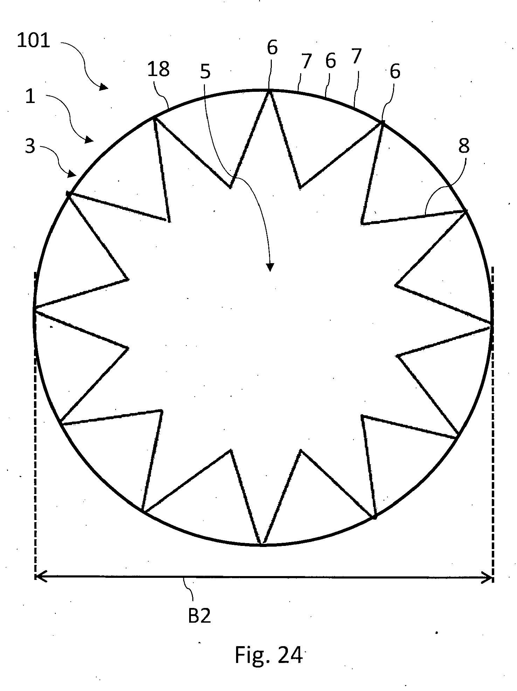

Figure 20 shows a top view of the stent 1 with magnets 6 in the deployed configuration 3 according to Fig. 19. The connecting elements 7 and magnets 6 form a closed loop 18 comprising a polygon structure arranged laterally outward of the temporary channel 5. The closed loops 18 in the distal region and proximal region of the stent 1 can apply pressure between the first and second portions of the apparatus 101 (not shown in Fig. 24). The shape of the closed loop 18 corresponds to the permanent compression anastomosis.The magnets are arranged at the maximum lateral distance of the stent 1. The shaping structure 8 of the stent 1 applies pressure laterally outward and contributes to anchoring the stent 1.

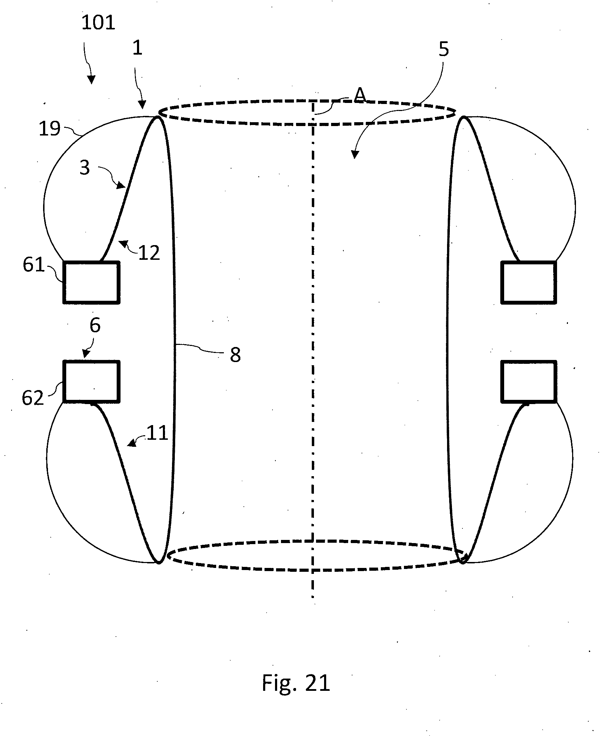

Figure 21 shows an embodiment of the stent 1 with magnets 6 according to Fig. 3. For references referring to aforementioned features refer to Fig. 3. The stent 1 in Fig. 21 comprises a protective cover 19 which renders the stent 1 atraumatic. The apparatus 101 can also be partially or completely covered with a smooth cover so that the stent can be guided along the gastrointestinal tract atraumatically and not get stuck. The dashed ellipses schematically illustrate the temporary channel 5.

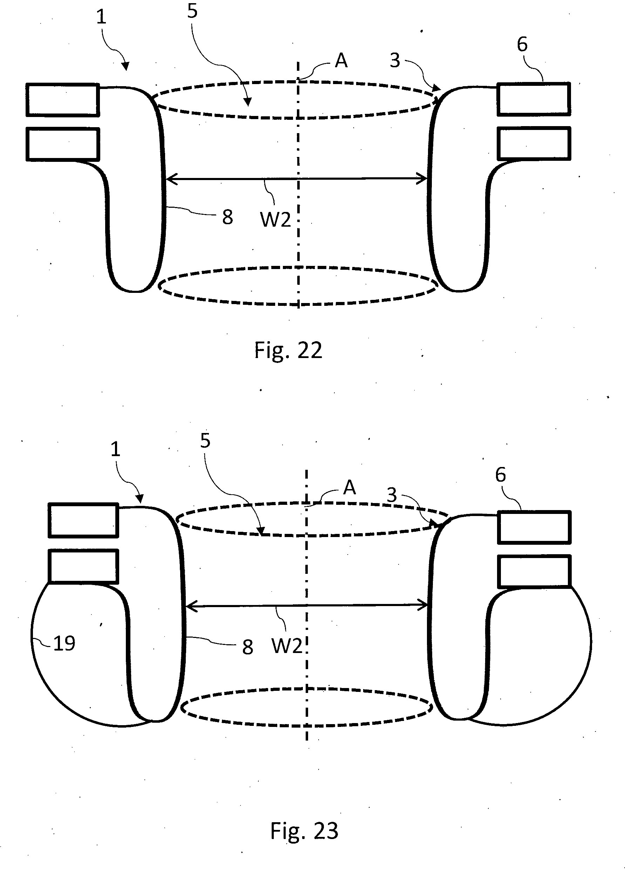

Figure 22 shows a cross-sectional view of an embodiment of the stent 1 with magnets 6 in a cross-sectional view forming a temporary channel 5 in the deployed configuration 3. The bent structure of the stent 1 in the embodiment of Fig. 22 prevents the stent 1 from being stuck or getting caught when detaching from the fused tissue walls. The majority of the shaping structure 8 of the stent 1 in the deployed configuration 3 is arranged in the distal region of the stent 1. The dashed ellipses schematically illustrate the diameter of the temporary channel 5.

Figure 23 shows a stent 1 according to Fig. 22 which comprises a single protective cover 19, in contrast to Fig. 21, which only has to be attached to the distal side of the stent 1 to render it atraumatic.

Figure 24 shows a top view of an alternative embodiment to Fig. 20 of a stent 1 with magnets 6 of the apparatus 101. For references relating to the same subject-matter please refer to Fig. 20. The lateral profile comprising of magnets 6 and connecting elements 7 and making up the closed loop 18 has a circular shape in contrast to a polygon shape.

Fig. 26 illustrates a further embodiment similar to Fig.. 24, but in which the stent 1 has an out-of-round shape, such as generally oval or generally elliptical, having a major transverse dimension B2. Such a stent may be useful for inducing non-round shapes of anastomosis. Also, the non-round shape may be more streamlined than a rounded shape, further facilitating evacuation of the stent 1 from the body after the stented-anastomosis has separated from the tissue wall sections.

In the illustrated embodiments the magnets are carried by and/or permanently attached to -the expandable stent. This enables the entire apparatus to be introduced in a single step, approaching the target site via a single route through the body, and without having to access both sides of the target site independently using two different devices via two different access routes.' However, a stent 1 without attached magnets 6 could also be conceived. The magnets 6 could be assembled to the stent 1 prior to introduction into the body. Alternatively, separate magnetic elements, e.g. magnetic rings, could be implanted in register with each other on respective tissue walls prior to, or following, implantation of the stent, the magnetic elements cooperating functionally with the stent as combined apparatus.

The foregoing description is merely illustrative of non-limiting examples useful for understanding the invention. Many modifications and equivalents may be used without departing from the principles of the invention. Although certain features and ideas have been highlighted above and in the appended claims, protection is claimed for any novel feature and/or idea described herein and/or illustrated in the drawings, whether or not emphasis has been placed thereon.

## Claims

1. Apparatus (101) for forming an anastomosis (201, 202) of two wall sections (4) of a patient's digestive system, optionally of the gastrointestinal tract, the apparatus having a non-deployed configuration for delivery to a target site for the anastomosis, and a deployed configuration for implantation, the apparatus comprising a stent (1) expandable from the non-deployed configuration to the deployed configuration for forming a stented anastomosis (201), the stent including a through-channel (5), and wherein in the deployed configuration of the apparatus, first and second portions (11, 12) of the apparatus are disposed laterally outside the through-channel in an annular arrangement, the first and second portions being magnetically attracted to each other for forming a compression anastomosis (202) around the through-channel of the stent.

2. Apparatus according to claim 1, wherein the stent (1) is self-expanding from the non-deployed configuration to the deployed configuration.

3. Apparatus according to claim 1 or 2, wherein, in addition to axial and/or lateral dimension changes, the stent (1) transforms in shape when expanding from the non-deployed configuration to the deployed configuration.

4. Apparatus according to claim 3, wherein at least one, optionally both, of the first and second portions (11, 12) is configured to move, when the apparatus expands from the non-deployed configuration to the deployed configuration, by:
a. swinging and/or articulating and/or bending laterally outwardly; and/or
b. moving from a position adjacent to an axial extremity of the stent when in the non-deployed position, to a position spaced from said axial extremity of the stent in the direction of an opposite axial extremity; and/or
c. moving closer to the other of the first and second portions.

5. Apparatus according to any preceding claim, wherein the through channel (5) of the stent is defined by a tubular wall (8) of the stent, and/or wherein the through-channel (5) of the stent expands in size laterally when the apparatus moves from the non-deployed configuration to the deployed configuration.

6. Apparatus according to any preceding claim, wherein at least one, optionally both, of the first and second portions (11, 12) comprises a plurality of magnets (6) arranged to permit the portion to transform between the non-deployed and deployed configurations of the apparatus, the magnets (6) arranged in an annular arrangement at least in the deployed configuration.

7. Apparatus according to claim 6, wherein at least some, optionally all, of the magnets (6) are fixedly attached to, and/or have a substantially fixed relation to, the stent (1) in the non-deployed and deployed configurations of the apparatus.

8. Apparatus according to claim 6, wherein at least one, optionally both, of the first and second portions (11, 12) comprises a closed-loop shaped pressure element (7) for pressing against the wall section tissue to induce tissue necrosis when the first and second portions (11, 12) are attracted magnetically, optionally wherein the pressing element (7) comprises a flexible filament, for example, a polymer filament.

9. Apparatus according to claim 6, 7 or 8, wherein the magnets (6) concertina in a circumference direction of the stent when in the non-deployed configuration of the apparatus.

10. Apparatus according to any preceding claim, wherein the apparatus changes configuration to a post-deployment configuration a period of time after implantation, the post-deployment configuration having a more streamlined shape than the deployed configuration for passing or removal through a body opening, for example, a natural body opening.

11. Apparatus according to claim 9, wherein at least one region of the stent (1) is configured to weaken and/or break a period of time after implantation, allowing the apparatus to transform from the deployed configuration to the post-deployed configuration.

12. Apparatus according to any preceding claim, wherein the stent (1) comprises or is made of one or more of: shape memory material; shape memory alloy; an alloy containing nickel and titanium; nitinol; stainless steel; tungsten; bioresorbable material.

13. Apparatus according to any preceding claim, wherein the stent (1) has one or more of: a self-expanding structure; a monolithic structure; a self-supporting structure; a polygon structure; a frame structure; a wire-mesh structure; one or more connectors between regions of the stent, at least one connector optionally made of bioresorbable material.

14. Apparatus according to any preceding claim, comprising an atraumatic cover of flexible material covering at least partly: (i) the stent and/or (ii) the first portion and/or (iii) the second portion.

15. A method for forming an anastomosis (202) of two sections of a patient's digestive system, optionally of the gastrointestinal tract, the method optionally using apparatus according to any of claims 1-14, comprising the steps of:
- insertion of the apparatus (101) into a patient, optionally through the oesophagus into the digestive system;
- piercing and/or'cutting through a first tissue wall (41) section and a second tissue wall (42) section creating two apertures at a target site for the anastomosis;
- optionally bringing the two intestine tissue wall (4) sections into close contact;
- introducing an expandable stent (1) to the target site;
- expanding the stent (1) from a non-deployed configuration (3) to a deployed configuration (2) forming a temporary stented anastomosis (201) between the wall sections, the stent having a through-channel to provide a functional anastomosis; and
- simultaneously or non-simultaneously with the expanding step, positioning first and second portions of the apparatus against tissue of the wall sections around the through-channel, the first and second portions magnetically attracting each other, for inducing a magnetic compression anastomosis (202).
